# EUROPEAN PATENT APPLICATION

(11) **EP 1 598 033 A1**
(43) Date of publication of application: **23.11.2005**
(21) Application number: 05010779.6
(22) Date of filing: 18.05.2005
(51) Int. Cl.: A61F 2/32, A61F 2/30, A61F 2/34

(54) **Prosthetic joint with annular contact bearing surface**

(30) Priority: 19.05.2004 US 849343
(71) Applicant: Howmedica Osteonics Corp., Mahwah, NJ 07430 (US)
(72) Inventor: Taylor, Scott K., 07450 Ridgewood, New Jersey (US)
(74) Representative: Wiebusch, Manfred

(57) **Abstract**

A prosthetic joint (50) has a head member (56) with a load-bearing surface (72), and a bearing member (60) with a concave bearing surface (70) oriented and configured such that during articulation of the prosthetic joint the load-bearing surface engages the concave bearing surface along an annular intermediate surface portion (74) located between first and second ends (64, 66) of the bearing member and spaced from each of the first and second ends. The configuration of the bearing surface provides clearance (80, 82) between the head member and the bearing surface along portions of the bearing surface lying between the annular intermediate surface portion and each end of the bearing member to assure engagement of the load-bearing surface at the annular intermediate surface portion. An opening (94) passes through the bearing member at the first end of the bearing member and communicates with the concave bearing surface.

## Description

The present invention relates generally to prosthetic implants and pertains, more specifically, to bearing members used in connection with head members in prosthetic joints, such as hip joints, in which a spherical head is engaged for articulation within a generally complementary bearing member.

The successful replacement of diseased or injured body joints, such as hip joints and shoulder joints, wherein a spherical head member is articulated within a bearing member, has led to the development of a very wide variety of prosthetic joints and surgical procedures which facilitate the replacement of such joints. More recently, it has been proposed that these prosthetic joints be constructed of materials which provide higher levels of performance, including improved articulation for greater comfort and increased range of motion, and improved resistance to wear for greater longevity.

The present invention provides a prosthetic joint construction having a unique geometry at the articular surfaces of the joint for realizing increased levels of performance. As such the present invention attains several objects and advantages, some of which are summarized as follows: promotes minimally invasive surgical procedures by providing a prosthetic joint with a profile configuration of reduced dimensions for enabling implant procedures utilizing smaller openings requiring incisions of minimal length and lower profile surgical instruments; enables the use of an annular bearing member having an apical opening which allows access to bone at an implant site subsequent to the implant of the bearing member at the site; deters dislocation of a head member from the bearing member of a prosthetic joint during service; allows an increased range of motion during articulation of a prosthetic joint, without deleterious impingement of a stem component on a cup component of the prosthetic joint; facilitates removal of the bearing member of a prosthetic joint, should such removal become necessary for replacement or revision; reduces any tendency toward pressure-induced osteolysis behind an implanted cup component of a prosthetic joint; assists in the natural lubrication of an implanted prosthetic joint; enables preservation of the round ligament (ligamentum capitus femoris or ligamentum teres) in a hip joint replacement; reduces cost and complexity in prosthetic joints; requires less bone removal, with a concomitant preservation of healthy bone, during joint replacement procedures; allows successful replacement of a natural joint at sites heretofore not amenable to the implant of conventional prosthetic joints; enables a wider choice of materials for the construction of prosthetic joints having increased levels of performance and longevity.

The above objects and advantages are attained by the present invention which may be described briefly as providing, in a prosthetic joint in which a head member is engaged with a bearing member for articulation within the prosthetic joint, the head member having a load-bearing surface for engaging the bearing member during articulation of the prosthetic joint, an improvement wherein the bearing member extends axially between a first end and a second end and includes a concave bearing surface having an orientation and a surface configuration arranged such that during articulation of the prosthetic joint, the load-bearing surface of the head member is engaged with the concave bearing surface along an annular intermediate surface portion located between the first and second ends of the bearing member, with the surface configuration of the concave bearing surface providing a first clearance between the head member and the concave bearing surface and extending in a first direction from the intermediate surface portion toward the first end of the bearing member, and a second clearance between the head member and the concave bearing surface and extending in a second direction from the intermediate surface portion toward the second end of the bearing member.

Further, the present invention provides, in a prosthetic joint in which a head member is engaged with a bearing member for articulation within the prosthetic joint, the head member having a longitudinal axis, circular surface contour configurations in planes transverse to the longitudinal axis, and a load-bearing surface with a predetermined radius extending from a given origin on the longitudinal axis for engaging the bearing member during articulation of the prosthetic joint, an improvement wherein: the bearing member extends axially between a first end and a second end, the bearing member including a concave surface extending axially between the first end and the second end and having an annular contact surface portion and a central axis for passing through the given origin when the head member is engaged with the bearing member; the concave surface having a surface contour configuration including circular profiles in radial planes transverse to the central axis, the circular profiles including a contact circular profile lying in a contact plane passing through the contact surface portion of the concave surface during articulation of the prosthetic joint, first circular profiles lying in respective first radial planes spaced axially from the contact plane and located between the contact plane and the first end of the bearing member, second circular profiles lying in respective second radial planes spaced axially from the contact plane and located between the contact plane and the second end of the bearing member, the contact circular profile having a prescribed contact radius, the first circular profiles each having a radius less than the prescribed contact radius and greater than a corresponding radius of a corresponding circular surface contour configuration of the head member, and the second circular profiles each having a radius greater than the prescribed contact radius and greater than a corresponding radius of a corresponding circular surface contour configuration of the head member; such that during articulation of the prosthetic joint, the load-bearing surface of the head member is engaged with the concave surface along the contact circular profile, intermediate the first and second ends of the bearing member, with the first circular profiles providing a first clearance between the head member and the surface contour configuration of the concave surface, and with the second circular profiles providing a second clearance between the head member and the surface contour configuration of the concave surface.

The invention will be understood more fully, while further objects and advantages will become apparent, in the following detailed description of preferred embodiments of the invention illustrated in the accompanying drawing, in which:
FIG. 1 is a partially diagrammatic longitudinal cross-sectional view of an acetabular component and a femoral component engaged therewith for articulation in a conventional prosthetic hip joint;
FIG. 2 is a partially diagrammatic longitudinal cross-sectional view of an acetabular component constructed in accordance with the present invention and a femoral component engaged therewith for articulation in a prosthetic hip joint;
FIG. 3 is a fragmentary diagrammatic depiction of the prosthetic hip joint of FIG. 2; and
FIG. 4 is an elevational, cross-sectional view showing an implanted prosthetic hip joint constructed in accordance with the present invention.

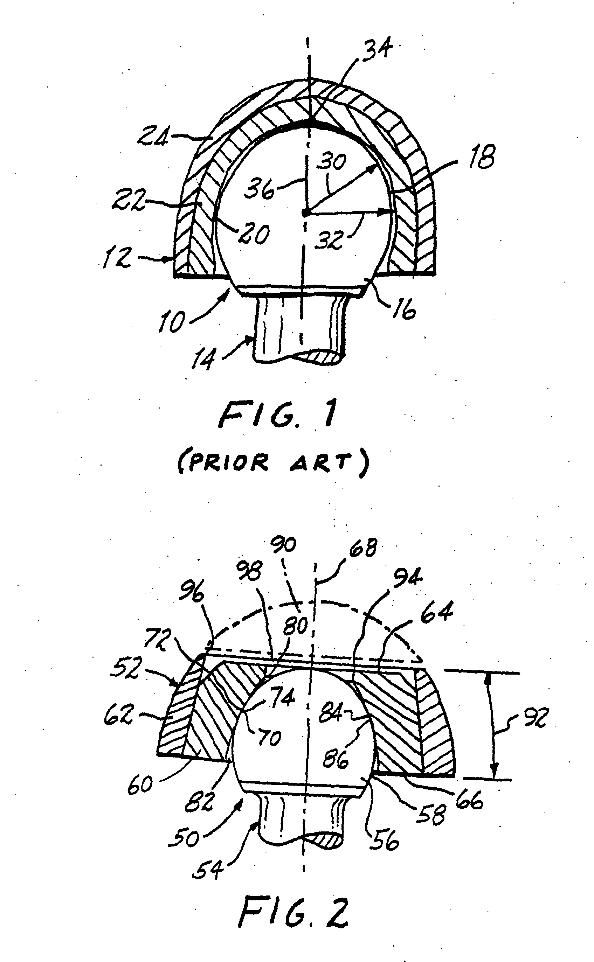

Referring now to the drawing, and especially to FIG. 1 thereof, a conventional prosthetic joint is shown in the form of prosthetic hip joint 10 and is seen to comprise an acetabular component 12 and a femoral component 14. Femoral component 14 includes a femoral head 16 having a spherical surface 18 which engages a generally complementary bearing surface 20 of a bearing member 22 secured within acetabular cup 24 of acetabular component 12 for articulation of the prosthetic hip joint 10, in a manner now well-known in the construction and operation of prosthetic joints.

Ideally, spherical surface 18 and bearing surface 20 would be made congruent for effective articulation of prosthetic hip joint 10; however, in order to avoid equatorial loading during articulation, as well as to meet the necessity for providing a range of sizes to accommodate various recipients of a prosthetic joint, as well as to compensate for manufacturing tolerances, the radius 30 of spherical surface 18 usually is made somewhat smaller than the radius 32 of the bearing surface 20. As a result, at least initial contact between the femoral head 16 and the bearing member 22 nominally is at a point 34 lying along a line 36 of load application. Deviations in the contour of bearing surface 20 in the vicinity of point 34 have been proposed in order to better distribute the load and reduce stresses at the load-bearing area of the bearing surface 20. Nevertheless, the load-bearing areas of the bearing surface 20 and of the spherical surface 18 remain juxtaposed with point 34.

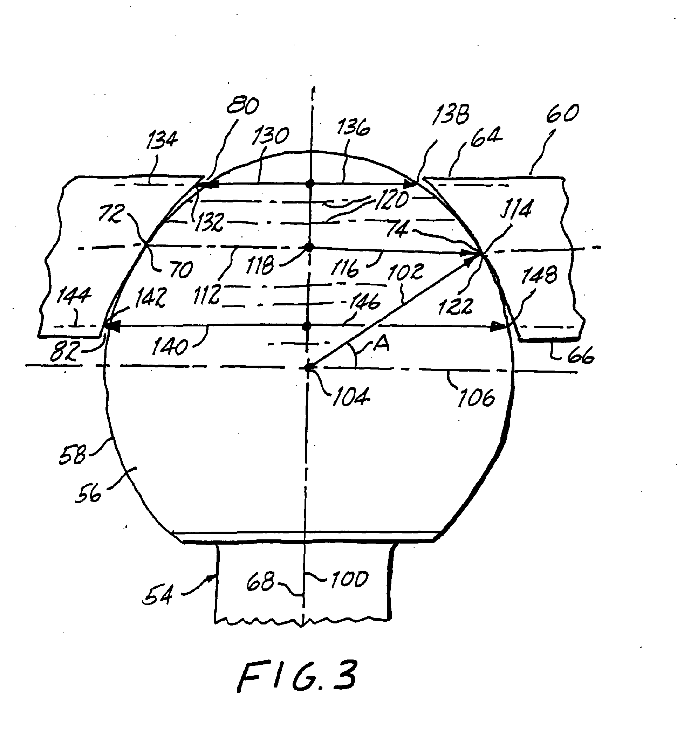

Turning now to FIG. 2, a prosthetic joint constructed in accordance with the present invention is shown in the form of prosthetic hip joint 50 comprised of an acetabular component 52 and a femoral component 54. As before, femoral component 54 includes a head member in the form of a femoral bead 56 having a spherical surface 58. A bearing member in the form of bearing 60 is affixed within an acetabular cup 62 and extends axially between a first, or upper end 64 and a second, or lower end 66, along a central axis 68. A bearing surface 70 within bearing 60 is oriented and configured such that during articulation of the prosthetic hip joint 50, a load-bearing surface 72 of femoral head 56 engages bearing surface 70 along an annular intermediate surface portion 74 of the bearing surface 70, the intermediate surface portion 74 being spaced from each of the upper and lower ends 64 and 66. Bearing surface 70 is concave and includes a surface profile configuration which provides a first, or upper clearance in the form of a proximal gap 80 between femoral head 56 and bearing surface 70 and a second, or lower clearance in the form of a distal gap 82 between femoral head 56 and bearing surface 70, the upper clearance extending in a first, or upward direction from intermediate surface portion 74 toward upper end 64 and the lower clearance extending in a second, or downward direction from intermediate surface portion 74 toward lower end 66. Thus, the surface profile configuration of bearing surface 70 assures that contact between spherical surface 58 of femoral head 56 and bearing surface 70 of bearing 60 lies along an annular seat 84 having an annular contact area 86 and that contact stresses are distributed over annular contact area 86 of annular seat 84.

The aspherical articulating geometry described above enables the distribution of contact stresses over annular contact area 86 of annular seat 84, resulting in a reduction of unit stress applied to the material of bearing 60. Further, by assuring that contact between femoral head 56 and bearing 60 is along an annular contact area 86 located between the ends 64 and 66 of the bearing 60, both the bearing 60 and the acetabular component 52 may be truncated, as compared to the configuration of conventional acetabular components. Thus, as illustrated in phantom in FIG. 2, apical portion 90 of a conventional acetabular component no longer need be present and acetabular component 52 is rendered more compact, with a reduced height 92 providing a lower profile configuration as compared to a conventional acetabular component. The lower profile configuration enables acetabular component 52 to be implanted with a surgical procedure which requires a smaller, minimal incision and lower profile surgical instruments, with a concomitant reduction in surgical trauma and convalescence. Further, less bone removal is required, enabling preservation of healthy bone at an implant site.

In addition, deletion of apical portion 90 provides an opening 94 at the upper end 64 of bearing 60, and a corresponding aperture 96 at the top of the acetabular component 52, enabling access to outer surfaces 98 of the acetabular cup 62, and to the acetabulum itself, subsequent to implant of the acetabular component 52 and insertion of the bearing 60 into the acetabular cup 62, without the necessity for interrupting the connection between the bearing 60 and the acetabular cup 62, and the possibility of compromising any locking mechanism which secures the bearing 60 in place in the acetabular cup 62. In this manner, a surgeon is able to make adjustments and corrections without disturbing the placement of the acetabular cup 62 at the acetabulum or the placement of the bearing 60 within the acetabular cup 62. Should it become necessary to remove the bearing 60 from the acetabular cup 62, removal is facilitated by the ability to grasp the bearing 60 at the opening 94, and such removal is accomplished readily without damage to either bearing 60 or acetabular cup 62.

Opening 94 and corresponding aperture 96 provide additional advantages in that the effective area for the transfer of fluid between the interior of the acetabular component 52 and the surrounding bone is increased, with a concomitant reduction in fluid pressures transferred to the acetabulum during service. The reduction of such fluid pressures avoids the creation of pressure-induced osteolysis behind the implanted acetabular component 52. In addition, fluid distribution to the articular surfaces is facilitated, thereby avoiding fluid starvation conditions during articulation, and promoting enhanced wear characteristics.

The aspherical articulating geometry of prosthetic hip joint 50 is illustrated diagrammatically in FIG. 3. Head 56 of femoral component 54 is engaged with bearing 60 of acetabular component 52 and includes a longitudinal axis 100 shown coincident with central axis 68 of bearing 60. Spherical surface 58 of head 56 has a predetermined radius 102 extending from a center 104 located in an equatorial plane 106 and placed on longitudinal axis 100, coincident with central axis 68, and is seated against bearing surface 70 at load-bearing surface 72. Spherical surface 58 includes circular surface contour configurations in planes transverse to longitudinal axis 100, one such plane being illustrated in the form of contact plane 112, within which contact plane 112 load-bearing surface 72 has a circular surface contour configuration 114 with a prescribed radius 116 extending from a given origin 118 on longitudinal axis 100 to the bearing surface 70.

As described above, load-bearing surface 72 contacts bearing surface 70 along a surface portion 74 spaced from each end 64 and 66 of bearing 60. Bearing surface 70 is concave and has a surface contour configuration which includes circular profiles 120 in radial planes transverse to central axis 68, one such circular profile being illustrated in the form of a contact circular profile 122 lying within contact plane 112, coincident with circular surface contour configuration 114 of spherical surface 58. Contact between load-bearing surface 72 and bearing surface 70 during articulation is along the annular intermediate surface portion 74, nominally along contact circular profile 122, within contact plane 112 spaced upwardly, in a proximal direction, from equatorial plane 106. The upward spacing between the equatorial plane 106 and the contact plane 112 is determined by an acute contact angle A between the equatorial plane 106 and the radius 102 of the spherical surface 58 which intercepts the contact plane 112 at the circular contact profile 122. The annular contact between head 56 and bearing 60 is spaced laterally from central axis 68 throughout articulation of the prosthetic joint 10 such that resultant load forces direct the femoral head 56 toward the acetabular component 52, thereby militating against dislocation of the head 56 from the bearing 60. Forces imposed by the load are distributed over the annular area of intermediate surface portion 74, rather than being concentrated in the vicinity of central axis 68, as would be the case with conventional prosthetic hip joint 10, thereby reducing unit stress along the bearing surface 70. Angle A is selected so as to optimize the attributes gained from the employment of an annular contact at intermediate surface portion 74. Thus, angle A may be within the range of about five to eighty-five degrees, with the preferred range being twenty to fifty degrees and a most-preferred nominal angle A being about thirty degrees.

Clearances provided by the proximal gap 80 and the distal gap 82 assure that the contact plane 112, and consequently contact between head 56 and bearing surface 70, occurs intermediate the ends 64 and 66 of bearing 60, thereby precluding excessive stresses at either end of the bearing surface 70 and the possibility of unwanted bearing failure or dislocation of the head 56 from the bearing 60. Gaps 80 and 82 are created by the relationship between spherical surface 58 and the surface contour configuration of bearing surface 70. More specifically, each gap 80 and 82 is established by a deviation between the contour of spherical surface 58 and the contour of bearing surface 70 at locations axially above and axially below the contact plane 112.

Looking first at the proximal gap 80, clearance between the spherical surface 58 and the bearing surface 70 is accomplished by a difference between the radius 130 of each circular profile 132 of the bearing surface 70 lying in each corresponding radial plane 134 spaced axially from the contact plane 112 and located between the contact plane 112 and the proximal end 64, and the radius 136 of a corresponding circular surface contour configuration 138 of the head 56. Contact plane 112 is located above equatorial plane 106 and the circular profiles 132 of the bearing surface 70 each have a radius 130 less than the prescribed contact radius 116. Looking next at the distal gap 82, clearance between the spherical surface 58 and the bearing surface 10 is accomplished by a difference between the radius 140 of each circular profile 142 of the bearing surface 70 lying in each corresponding radial plane 144 spaced axially from the contact plane 112 and located between the contact plane 112 and the distal end 66, and the radius 146 of a corresponding circular surface contour configuration 148 of the head 56. Contact plane 112 is located above equatorial plane 106 and the circular profiles 142 of the bearing surface 70 each have a radius 140 greater than the prescribed contact radius 116.

The magnitude of each gap 80 and 82 is selected to accommodate different conditions encountered at the site of the implant, as well as to enable a reduction in the dimensions of the bearing 60. Gaps 80 and 82 accommodate different fluid conditions at the implant site and allow for the egress of any particles which may be generated by wear. The clearance provided by gaps 80 and 82, as measured radially between the spherical surface 58 and the bearing surface 70, may be in the range of ten to two-thousand microns, with the preferred range being twenty to two-hundred microns and the most-preferred clearance nominally being forty microns.

While the spherical bearings employed in conventional prosthetic joints require a relatively broad hemispherical area in order to accommodate a contact point which moves during articulation, the location of the annular contact provided by the aspherical geometry described above remains essentially unchanged during articulation. Thus, the amount of material needed to support the load applied during articulation is a function only of the strength of the material and lower profiles are attained in prosthetic hip joint 50 by deleting excess material at the distal end of the bearing 60. Both the contact angle A and the radial clearance provided at gaps 80 and 82 are selected for a reduction in the profile dimensions of the bearing 60. The aspherical geometry is produced readily by conventional machining techniques and can be molded of various bearing materials. Hard materials are better choices, with ceramics being preferred over metals and composite materials. Both monolithic and modular prosthetic joints can incorporate the aspherical geometry described herein.

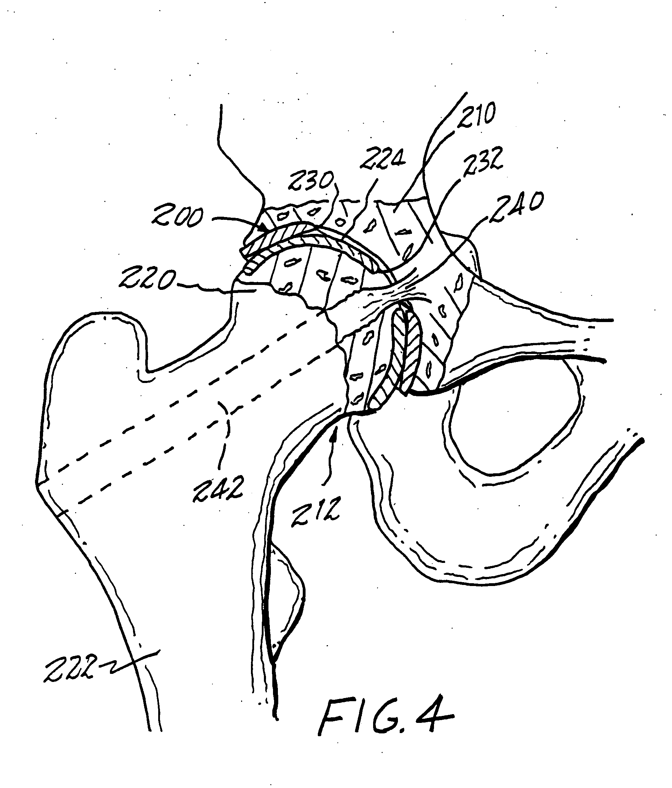

Referring now to FIG. 4, a truncated, annular acetabular component 200 constructed in accordance with the present invention is shown implanted within the natural acetabulum 210 of a hip joint 212. The natural femoral head 220 of femur 222 has been restored with a spherical surface replacement component 224. The acetabular component 200 is provided with an opening 230, as described above in connection with acetabular component 52, and a corresponding aperture 232 is provided in the replacement component 224. In this manner, the round ligament 240 (ligamentum capitus femoris, or ligamentum teres) is preserved, thereby avoiding sacrifice of the round ligament 240. The conduit for the supply of blood to the femoral head 220 is preserved. Access to ligament 240 for detachment and subsequent reconstruction and re-attachment is shown at 242. In addition, preservation or even reconstruction of the round ligament 240 assists in resisting femoral head dislocation under certain hip movements, thereby reducing the incidence of dislocation. Further, the construction of the truncated acetabular component 200 allows for the use of a synthetic ligament.

It will be seen that the present invention attains all of the objects and advantages summarized above, namely: promotes minimally invasive surgical procedures by providing a prosthetic joint with a profile configuration of reduced dimensions for enabling implant procedures utilizing smaller openings requiring incisions of minimal length and lower profile surgical instruments; enables the use of an annular bearing member having an apical opening which allows access to bone at an implant site subsequent to the implant of the bearing member at the site; deters dislocation of a head member from the bearing member of a prosthetic joint during service; allows an increased range of motion during articulation of a prosthetic joint, without deleterious impingement of a stem component on a cup component of the prosthetic joint; facilitates removal of the bearing member of a prosthetic joint, should such removal become necessary for replacement or revision; reduces any tendency toward pressure-induced osteolysis behind an implanted cup component of a prosthetic joint; assists in the natural lubrication of an implanted prosthetic joint; enables preservation of the round ligament (ligamentum capitus femoris or ligamentum teres) in a hip joint replacement; reduces cost and complexity in prosthetic joints; requires less bone removal, with a concomitant preservation of healthy bone, during joint replacement procedures; allows successful replacement of a natural joint at sites heretofore not amenable to the implant of conventional prosthetic joints; enables a wider choice of materials for the construction of prosthetic joints having increased levels of performance and longevity.

It is to be understood that the above detailed description of preferred embodiments of the invention is provided by way of example only. Various details of design, construction and procedure may be modified without departing from the true spirit and scope of the present invention, as set forth in the appended claims.

## Claims

1. A prosthetic joint (50; 212), in which a head member (56; 224) is engaged with a bearing member (60; 200) for articulation within the prosthetic joint, the head member having a load-bearing surface (72) for engaging the bearing member during articulation of the prosthetic joint, and wherein the bearing member extends axially between a first end (64) and a second end (66) and includes a concave bearing surface (70) having an orientation and a surface configuration arranged such that during articulation of the prosthetic joint, the load-bearing surface (72) of the head member is engaged with the concave bearing surface (70) along an annular intermediate surface portion located between the first and second ends of the bearing member, with the surface configuration of the concave bearing surface providing a first clearance (80) between the head member and the concave bearing surface and extending in a first direction from the intermediate surface portion (74) toward the first end (64) of the bearing member, and a second clearance (82) between the head member and the concave bearing surface and extending in a second direction from the intermediate surface portion toward the second end (66) of the bearing member.

2. The joint of claim 1, wherein:
the head member has a longitudinal axis (68), circular surface contour configurations (138, 140) in planes transverse to the longitudinal axis, and the load-bearing surface (72) has a predetermined radius (102) extending from a given origin (104) on the longitudinal axis for engaging the bearing member during articulation of the prosthetic joint,
the concave surface (70) of the bearing member (60, 200) extends axially between the first end (64) and the second end (66) and has an annular contact surface portion (74) and a central axis for passing through the given origin (104) when the head member is engaged with the bearing member;
the concave surface (70) has a surface contour configuration including circular profiles (120) in radial planes transverse to the central axis, the circular profiles including
a contact circular profile (122) lying in a contact plane (112) passing through the contact surface portion of the concave surface during articulation of the prosthetic joint,
first circular profiles (132) lying in respective first radial planes (136) spaced axially from the contact plane (112) and located between the contact plane and the first end (64) of the bearing member,
second circular profiles (142) lying in respective second radial planes (146) spaced axially from the contact plane (112) and located between the contact plane and the second end (66) of the bearing member,
the contact circular profile (122) having a prescribed contact radius (116),
the first circular profiles (132) each having a radius (130) less than the prescribed contact radius and greater than a corresponding radius of a corresponding circular surface contour configuration (138) of the head member, and
the second circular profiles (142) each having a radius (140) greater than the prescribed contact radius and greater than a corresponding radius of a corresponding circular surface contour configuration (148) of the head member;
such that during articulation of the prosthetic joint, the load-bearing surface (72) of the head member is engaged with the concave surface (70) along the contact circular profile (122), intermediate the first and second ends of the bearing member, with the first circular profiles providing the first clearance (80) between the head member and the surface contour configuration of the concave surface, and with the second circular profiles providing the second clearance (82) between the head member and the surface contour configuration of the concave surface.

3. The joint of claim 1 or 2, wherein the bearing member (60) includes an opening (94) at the first end (64), the opening extending axially and communicating with the concave surface (70).

4. The joint of claim 1, 2 or 3, wherein the head member (56; 224) includes a spherical surface (58) and the annular contact surface portion and the circular surface contour configurations (138, 140) are located on the spherical surface.

5. The joint of claim 4 wherein the prosthetic joint comprises a prosthetic hip joint (50; 212), the bearing member (60) comprises an acetabular bearing, the first end (64) comprises a proximal end of the bearing member and the second end (66) comprises a distal end of the bearing member.

6. The joint of claim 4 or 5 wherein the spherical surface (58) has a predetermined radius (102) extending between a center (104) and intermediate surface portion (74), the center being placed in an equatorial plane (106) of the head member and located on the central axis (68) of the concave surface when the head member is engaged with the bearing member, the predetermined radius making an acute angle (A) with the equatorial plane such that the contact plane (112) is located between the equatorial plane (106) and the first end (64) of the bearing member.

7. The joint of claim 6, wherein the acute angle (A) is in a range of about twenty to fifty degrees.

8. The joint of claim 7, wherein the acute angle (A) is about thirty degrees.

9. The joint of any of the claims 1 to 8, wherein the first clearance (80) is in a range of about twenty to two-hundred micrometers.

10. The joint of claim 9, wherein the first clearance (80) is about forty micrometers.

11. The joint any of the claims 1 to 10, wherein the second clearance (82) is in a range of about twenty to two-hundred micrometers.

12. The joint of claim 11 wherein the second clearance (82) is about forty micrometers.
